(19) Europäisches Patentamt
European Patent Office
Office européen des brevets

(11) Veröffentlichungsnummer: **0 175 264**
**B1**

(12) **EUROPÄISCHE PATENTSCHRIFT**

(45) Veröffentlichungstag der Patentschrift: **15.11.89**

(51) Int. Cl.⁴: **C 07 D 403/04**, C 07 D 241/20, C 07 D 241/26

(21) Anmeldenummer: **85111443.9**

(22) Anmeldetag: **10.09.85**

# (54) Verfahren zur Herstellung von 2-Amino-3-cyano-5-dialkoxymethyl-pyrazinen und Zwischenprodukte für dieses Verfahren.

(30) Priorität: **15.09.84 DE 3433960**

(43) Veröffentlichungstag der Anmeldung: **26.03.86 Patentblatt 86/13**

(45) Bekanntmachung des Hinweises auf die Patenterteilung: **15.11.89 Patentblatt 89/46**

(84) Benannte Vertragsstaaten: **CH DE FR GB LI NL**

(56) Entgegenhaltungen: **US-A-4 267 327**

(73) Patentinhaber: **BASF Aktiengesellschaft, Carl-Bosch- Strasse 38, D-6700 Ludwigshafen (DE)**

(72) Erfinder: **Leininger, Hartmut, Dr., Siebenpfeiffer-Strasse 22, D-6730 Neustadt (DE)**
Erfinder: **Littmann, Wolfgang, Dr., Neckarpromenade 36, D-6800 Mannheim 1 (DE)**
Erfinder: **Paust, Joachim, Dr., Ringstrasse 3, D-6706 Neuhofen (DE)**

**EP 0 175 264 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft ein Verfahren zur Herstellung von 2-Amino-3-cyano-5-dialkoxymethyl-pyrazin-derivaten sowie in 3- und 5-Stellung substituierte 2-Amino-pyrazin-derivate der allgemeinen Formel I

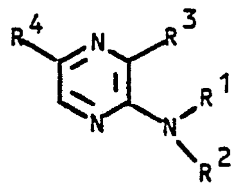

(I),

in der $R^1$ und $R^2$ für Wasserstoff oder eine für Aminogruppen übliche Schutzgruppe stehen, wobei $R^1$ und $R^2$ auch miteinander verbunden sein können,

$R^3$ für eine der Gruppen $-CH_2-O-CO-CH_3$ (a), $-CH_2OH$ (b),

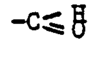

(c), oder $-C \equiv N$ (d) steht und

$R^4$ für $-CHCl_2$ oder $-CHBr_2$ steht oder

$R^4$ für den Fall, daß $R^1$ und $R^2$ für eine Schutzgruppe stehen,

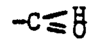 be-

bedeutet, als neue Zwischenprodukte für dieses Verfahren.

Der Erfindung lag die allgemeine Aufgabe zugrunde, die physiologisch wichtige Folsäure

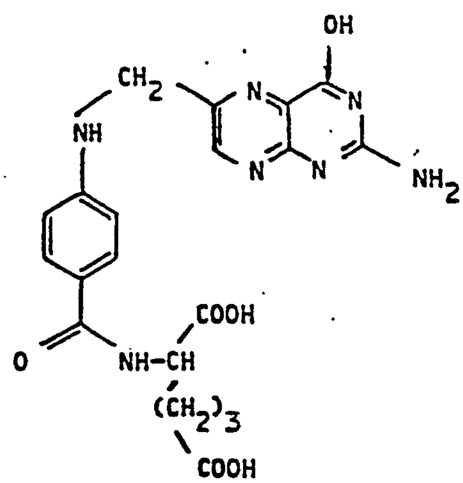

sowie deren Derivate besser zugänglich zu machen. Speziell betraf die Aufgabe neue Zwischenprodukte mit der 2-Amino-pyrazin-Struktur, die einerseits gut zugänglich sind und sich andererseits auf relativ einfache Weise in reaktionsfähige Pteridine und weiter in die Folsäure bzw. deren Derivate überführen lassen.

Aus einer Arbeit von E. C. Taylor et al. (J. Org. Chem., Vol. 43 No. 4 (1978) Seiten 736 - 37) ist es bekannt, daß man 6-Formyl-pterin der Formel

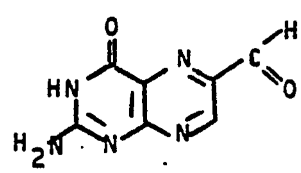

,

welches eine Schlüsselverbindung für Folsäure und die verschiedensten Derivate davon darstellt, auf relativ einfache Weise und mit guten Ausbeuten aus 2-Amino-3-cyano-5-dialkoxymethyl-pyrazin herstellen kann. Die gemäß dieser Arbeit für die Herstellung des 2-Amino-3-cyano-5-dialkoxymethyl-pyrazins vorgesehene Ausgangsverbindung, das 2-Amino-3-cyano-5-chlormethyl-pyrazin ist jedoch leider selbst nicht gut zugänglich. Man erhält es beispielsweise in nur mäßigen Ausbeuten aus Aminomalonitril und β-Chloro-pyruvaldoxim,

welches seinerseits aus Keten hergestellt werden kann und eine physiologisch äußerst unangenehme Substanz ist, die sich bei Raumtemperatur unter Bildung von Blausäure zersetzt und daher für eine technische Umzetzung ungeeignet ist (vgl. E. C. Taylor et al. J. Org. Chem. 38 (1973), 806). Trotz großer Anstrengungen des Arbeitskreises um E. C. Taylor (vgl. J. Org. Chem. 45 (1980) Seiten 2485 - 2489 und J. Org. Chem. 46 (1981), Seiten 1394 - 1402) ist es nicht gelungen, ein technisch brauchbares Verfahren zur Herstellung von reinen, isomerenfreien 2-Amino-3-cyano-5-dialkoxymethyl-pyrazinen zu entwickeln, so daß die 2-Amino-3-cyano-5-dialkoxymethyl-pyrazine bislang als nur schwer zugängliche, in schlechten Ausbeuten, in zahlreichen Stufen und/oder aus teuren und nicht gut synthetisierbaren Ausgangsmaterialien herstellbare Substanzen bekannt waren.

Es war daher die Aufgabe der Erfindung, neue Zwischenprodukte mit der 2-Amino-pyrazin-Struktur zu finden, die einerseits gut zugänglich sind und andererseits auf relativ einfache Weise in 2-Amino-3-cyano-5-dialkoxymethyl-pyrazin überführt werden können, wodurch ein vorteilhaftes Gesamtverfahren zur Herstellung von Folsäure und davon abgeleiteten Verbindungen über 6-Formyl-pterin ermöglicht würde.

Es wurde nun gefunden, daß man 2-Amino-3-cyano-5-dialkoxymethyl-pyrazine ausgehend von neuen 3-Halogenmethyl-5-dihalogenmethyl-2-amino-pyrazin-derivaten über die neuen in 3- und 5-Stellung substituierten 2-Amino-pyrazin-derivate der allgemeinen Formel I, insbesondere über die in 3- und 5-Stellung substituierten 2-Phthalimido-pyrazine der allgemeinen Formel II

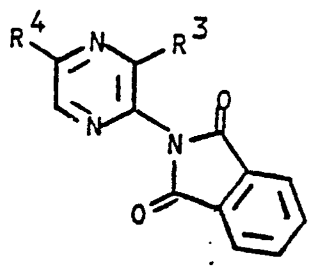

(II),

das sind 2-Amino-pyrazinderivate der allgemeinen Formel I, in der $R^1$ und $R^2$ zusammen für die Gruppe

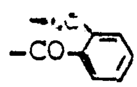

stehen,

$R^3$ für eine der Gruppen $-CH_2-O-CO-CH_3$ (a), $-CH_2OH$ (b),

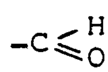

(c), oder $-C{\equiv}N$ (d) steht und

$R^4$ für $-CHCl_2$, $-CHBr_2$ oder

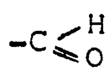

steht,

überraschend vorteilhaft erhält.

Gegenstand der Erfindung ist daher neben den neuen Zwischenprodukten der allgemeinen Formel I und insbesondere der allgemeinen Formel II ein Verfahren zur Erstellung von 2-Amino-3-cyano-5-dialkoxymethyl-pyrazinderivaten der allgemeinen Formel III

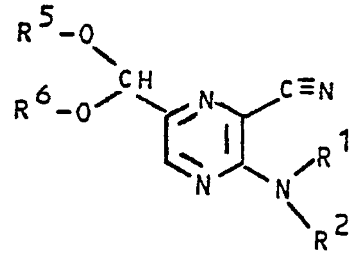

(III),

in der $R^1$ und $R^2$ für Wasserstoff oder eine für Aminogruppen übliche Schutzgruppe stehen, wobei $R^1$ und $R^2$ auch miteinander verbunden sein können, insbesondere $R^1$ und $R^2$ zusammen für die Gruppe

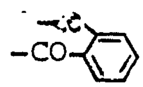

stehen
und $R^5$ und $R^6$ für eine Alkylgruppe mit 1 bis 4 C-Atomen stehen, oder aber $R^5$ und $R^6$ zusammen für einen gegebenenfalls durch Methylgruppen substituierten Ethylen- oder Propylenrest stehen, das dadurch gekennzeichnet ist, daß man

A. ein neues 3-Halogenmethyl-5-dihalogenmethyl-2-amino-pyrazin-derivat der allgemeinen Formel IV

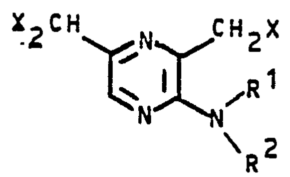

(IV),

in der X für Cl oder Br vorzugsweise Cl steht, entweder direkt oder über die neue 3-Acetoxymethyl-5-dihalogenmethyl-2-amino-pyrazin-Verbindung der allgemeinen Formel Ia

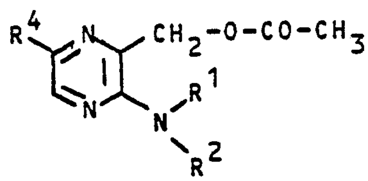

(Ia),

in der $R^1$ und $R^2$ die oben angegebene Bedeutung haben und $R^4$ für $-CHCl_2$ oder $-CHBr_2$, vorzugsweise für $-CHCl_2$ steht,
in das neue 3-Hydroxymethyl-5-dihalogenmethyl-2-amino-pyrazin-derivat der allgemeinen Formel Ib überführt,

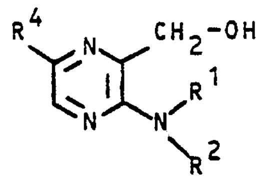

(Ib)

B. dieses zu dem entsprechenden neuen 3-Formyl-5-dihalogenmethyl-2-amino-pyrazin-derivat der allgemeinen Formel Ic oxidiert

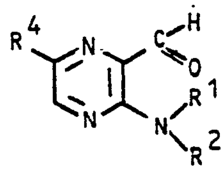

(Ic),

C. dieses in an sich bekannter Weise in das entsprechende neue 3-Cyano-5-dihalogenmethyl-2-amino-pyrazin-derivat der allgemeinen Formel Id überführt,

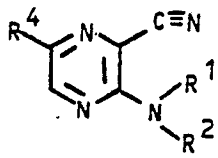

(Id),

D. dieses in an sich bekannter Weise in das entsprechende 3-Cyano-5-formyl-2-amino-pyrazin-derivat der allgemeinen Formel Ie überführt

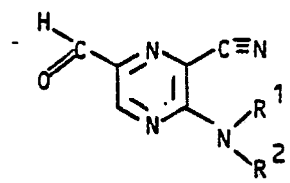

(Ie),

und

E. dieses acetalisiert und gegebenenfalls die Schutzgruppe von der Aminogruppe abspaltet.

Ganz besonders vorteilhaft gestaltet sich das Verfahren, wenn man 3-Chlormethyl-5-dichlormethyl-2-phthalimidopyrazin (IVf) als Ausgangsmaterial verwendet.

Im Hinblick auf den Stand der Technik ist es sehr überraschend, daß die Verbindungen der Formel IV, insbesondere IVf, in sehr einfachen Reaktionsschritten bei hohen chemischen Ausbeuten und ohne Benutzung kostspieliger und aufwendiger Chemikalien glatt zu den gewünschten 2-Amino-3-cyano-5-dialkoxymethyl-pyrazinen der Formel III umgesetzt werden können.

Die neuen Ausgangsstoffe der allgemeinen Formel IV können in relativ guten Ausbeuten gemäß einem gleichzeitig zum Patent angemeldeten Verfahren (vgl. Patentanmeldung EP-A-175 263 durch Halogenierung der entsprechenden 2-Amino-3,5-dimethyl-pyrazine hergestellt werden, welche ihrerseits beispielsweise durch Cyclisierung von α-Imino-dipropionsäurenitril der Formel V mit Halogenwasserstoffen

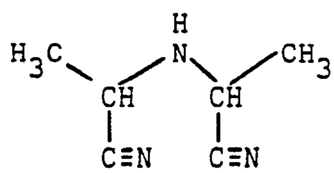

(V)

vorteilhaft erhältlich sind. Letztere kann z. B. durch die in DE-OS-1 493 752 beschriebene Umsetzung von Ammoniak mit Acetaldehyd und Blausäure erhalten werden.

Das bei den genannten Verfahren anfallende Halogenierungsproduktgemisch enthält das gewünschte Isomere bei entsprechender Verfahrensführung in einem Prozentanteil von 60 bis 70 % und kann als solches eingesetzt werden.

**Reaktionsschritt A**

Die Umsetzung der Verbindungen der Formel IV zu den entsprechenden 3-Hydroxymethylverbindungen Ib kann in einem Schritt oder aber über die neuen 3-Acetoxy-Verbindungen Ia erfolgen. Zur Herstellung über die 3-Acetoxy-Verbindungen Ia werden die Verbindungen IV in einem polaren organischen Lösungsmittel, wie Dimethylformamid oder insbesondere Dimethylsulfoxid gelöst und in Gegenwart von Eisessig mit einem

Alkaliacetat unter milden Bedingungen - beispielsweise durch längeres Rühren bei Raumtemperatur (RT) in guten Ausbeuten umgesetzt. Das erhaltene Produkt kann auf übliche Weise problemlos isoliert und gereinigt werden und anschließend in einer Umesterungsreaktion mit einem niedrig siedenden Alkohol, wie Methanol, in Gegenwart eines sauren Katalysators, wie p-Toluol-sulfonsäure unter Abdestillieren des gebildeten Essigsäuremethylesters in die entsprechenden 3-Hydroxymethyl-Verbindungen Ib überführt werden. Setzt man dem beschriebenen, aus IV, dem Lösungsmittel, Eisessig und Alkaliacetat bestehenden Reaktionsgemisch Wasser zu - beispielsweise in Mengen von 3 bis 20, vorteilhaft etwa 5 %, bezogen auf das Reaktionsgemisch, so erhält man unter sonst etwa gleichen Bedingungen direkt die 3-Hydroxymethyl-Verbindung Ib in Ausbeuten von ca. 50 % nach Umkristallisation. Der so erhaltene Alkohol kann auf übliche Weise, z. B. durch Extraktion, bequem isoliert und gereinigt werden.

Es war überraschend, daß dieser Schritt so vorteilhaft durchgeführt werden kann, insbesondere aber, daß diese Umsetzung auch in nur einem Reaktionsschritt mit relativ guten Ausbeuten erfolgt.

**Reaktionsschritt B**

Die Oxidation der 3-Hydroxymethyl-Verbindungen Ib zu den neuen 3-Formyl-Verbindungen Ic muß mit einem Oxidationsmittel, das ausschließlich die Hydroxymethylgruppe zur Formylgruppe oxidiert, in einem gegen dieses Oxidationsmittel unempfindlichen Lösungsmittel erfolgen. Überraschend vorteilhaft gelingt die Reaktion mit unter milden Bedingungen oxidierenden Agentien, wie dem Pyridiniumchlorochromat in Halogenkohlenwasserstoffen, wie Dichlormethan als Lösungsmittel. Nähere Einzelheiten über diese Oxidationsmethode finden sich in Tetrahedron letters 1975, 2647.

**Reaktionsschritt C**

Die Umwandlung der 3-Formylverbindungen Ic in die entsprechenden 3-Cyano-Verbindungen Id erfolgt in an sich bekannter Weise, so daß sich eine ausführliche Beschreibung des Verfahrens erübrigt. Besonders einfach und problemlos gelingt die Umsetzung, indem man Ic zunächst in einem polaren Lösungsmittel, wie Dimethylsulfoxid, mit einem Hydroxlammoniumsalz umsetzt, anschließend die Oximinogruppe mit üblichen Acylierungsmitteln, wie Acetanhydrid verestert und dann die die Acetoximinogruppe enthaltende Verbindung in einem höhersiedenden Lösungsmittel thermisch zersetzt. Auch diese Umsetzung gelingt mit überraschend guten Ausbeuten.

**Reaktionsschritte D und E**

Die Umwandlung der 5-Dihalogenmethyl-3-cyano-Verbindungen Id über die 5-Formyl-3-cyano-Verbindungen Ie in die 5-Dialkoxymethyl-pyrazin-derivate III erfolgt in an sich bekannter Weise, so daß sich detaillierte Angaben erübrigen.

Das erfindungsgemäße Verfahren und die neuen Zwischenprodukte der Formel I ermöglichen erstmals einen auch technisch gangbaren Reaktionsweg zu 2-Amino-3-cyano-5-dialkoxymethyl-pyrazinen und dadurch zu 6-Formylpterin, einer Schlüsselverbindung für Folsäure und davon abgeleitete Wirkstoffe.

**Beispiel 1**

a) Herstellung von 3-Acetoxymethyl-5-dichlormethyl-2-phthalimido-pyrazin

Eine Lösung von 57,7 g (0,16 mol) 3-Chlormethyl-5-dichlormethyl-2-phthalimido-pyrazin (65 %-ig) in 1000 ml Dimethylsulfoxid wurde mit 50 ml absolutem Eisessig und 35 g Kaliumacetat versetzt und dann drei Tage bei RT gerührt. Anschließend wurde das Reaktionsgemisch mit 1000 ml Dichlormethan und 1000 ml Eiswasser versetzt, durchmischt und eine Phasentrennung durchgeführt. Die Wasserphase wurde einmal mit 500 ml und einmal mit 250 ml Dichlormethan extrahiert. Die vereinigten organischen Phasen wurden zweimal mit 1000 ml Wasser gewaschen, über Natriumsulfat getrocknet und am Rotationsverdampfer eingeengt. Das schwarzbraune Öl wurde in 20 ml Methanol in der Siedehitze aufgenommen und bei RT auskristallisieren lassen. Anschließend wurde in wenig Diethylether aufgeschlämmt, filtriert und getrocknet. Die Kristalle wurden dann erneut aus Methanol umkristallisiert. Man erhielt 30,6 g (77 % der Theorie) an 3-Acetoxymethyl-5-dichlormethyl-2-phthalimidopyrazin mit einem Festpunkt F = 117 bis 118°C.

$^1$H-NMR (270 MHz, $CDCl_3$): δ 1,9 (s, 3H), 5,3 (s, 2H), 6,9 (s, 1H), 8,1 (m, 4H), 9,1 (s, 1H).

b) Herstellung von 3-Hydroxymethyl-5-dichlormethyl-2-phthalimido-pyrazin

Eine Mischung von 15 g (0,04 mol) 3-Acetoxymethyl-5-dichlormethyl-2-phthalimido-pyrazin, 150 ml Methanol und 0,15 g p-Toluolsulfonsäure wurde zum Sieden erhitzt und hieraus langsam innerhalb von 5 Stunden ein Gemisch aus Methanol und Essigsäuremethylester abdestilliert. Dann wurde auf ein Drittel des Gesamtvolumens eingeengt und die ausgefallenen Kristalle isoliert. Die Umkristallisation erfolgte aus Essigsäureethylester. Man erhielt 7,18 g (54 %) 5-Dichlormethyl-3-hydroxymethyl-2-phthalimido-pyrazin vom Festpunkt F = 188 bis 189°C.
$^1$H-NMR ($CDCl_3$ 270 MHz,): 4,7 (s, 2H), 6,9 (s, 1H), 8,1 (m, 4H), 9,1 (s, 1H).

c) Herstellung von 3-Formyl-5-dichlormethyl-2-phthalimido-pyrazin

Eine Lösung von 4 g 3-Hydroxymethyl-5-dichlormethyl-2-phthalimido-pyrazin in 80 ml Dichlormethan wurde bei 35°C portionsweise mit 5,1 g Pyridiniumchlorochromat versetzt, dann 40 Minuten gerührt, über Kieselgel filtriert und das Filtrat eingeengt. Man erhielt 3,1 g (76 %) farblose, bei 145 bis 146°C schmelzende Kristalle (aus Essigester) von 3-Formyl-5-dichlormethyl-2-phthalimido-pyrazin.
$^1$H-NMR (270 MHz, $CDCl_3$): 6,9 (s, 1H), 8,0 (m, 4H), 9,3 (s, 1H), 10,1 (s, 1H).

d) Herstellung von 3-Oximino-5-dichlormethyl-2-phthalimido-pyrazin
Eine Lösung von 10 g 3-Formyl-5-dichlormethyl-2-phthalimido-pyrazin in 50 ml Dimethylsulfoxid wurde auf 50°C erwärmt, mit 7 g Hydroxylaminhydrochlorid versetzt und 15 Min. bei 70°C gerührt. Anschließend wurde die Reaktionslösung mit 700 ml Wasser versetzt und filtriert und das Filtrat mit wenig Diethylether nachgewaschen. Man erhielt 6,8 g farblose Kristalle (65 %-ig) von 3-Oximino-dichlormethyl-2-phthalimido-pyrazin.
$^1$H-NMR (270 MHz, DMSO-d6): δ 7,7 (s, 1H), 8,0 (m, 4H), 8,3 (s, 1H), 9,1 (s, 1H), 12,0 (s, OH).

e) Herstellung von 3-Acetoximino-5-dichlormethyl-2-phthalimido-pyrazin

Eine Suspension von 10 g 3-Oximino-5-dichlormethyl-2-phthalimido-pyrazin in 15 ml Acetanhydrid wurde auf 100°C erhitzt, wobei die Suspension zu einer klaren Lösung wurde. Anschließend wurde noch weitere 15 Min. bei 100°C belassen und dann das Reaktionsgemisch unter vermindertem Druck zur Trockene eingeengt. Der erhaltene Rückstand wurde aus absolutem Methanol umkristallisiert. Man erhielt 10 g 3-Acetoximino-5-dichlormethyl-2-phthalimido-pyrazin.
$^1$H-NMR (270 MHz, DMSO-$d_6$): 1,9 und 2,1 (jeweils s, 3H), 7,8 (s, 1H), 8,1 (m, 4H), 9,0 und 9,3 (jeweils s, 1H), 9,5 (s, 1H).

f) Herstellung von 3-Cyano-5-dichlormethyl-2-phthalimido-pyrazin

Eine Lösung von 10 g 3-Acetoximino-5-dichlormethyl-2-phthalimido-pyrazin wurde in 100 ml 1,2-Dichlorbenzol 60 Min. unter Rückfluß zum Sieden erhitzt. Dann destillierte man das Lösungsmittel bei $10^{-2}$ mbar ab, nahm den Rückstand in wenig absolutem Methanol auf und saugte ab. Man erhielt 8,1 g (95 %) 3-Cyano-5-dichlormethyl-2-phthalimido-pyrazin.

g) Herstellung von 3-Cyano-5-dimethoxymethyl-2-phthalimido-pyrazin

Zu einer Lösung von 3 g 3-Cyano-5-dichlormethyl-2-phthalimido-pyrazin in 60 ml Dichlormethan wurden vorsichtig 5 ml Morpholin zugetropft und das Reaktionsgemisch eine Stunde unter schwachem Sieden unter Rückfluß gehalten. Anschließend wurde im Eisbad abgekühlt und abgesaugt. Die organische Phase wurde eingeengt, der Rückstand in Methanol aufgenommen und mit 2 g aktiviertem saurem Ionenaustauscher versetzt. Anschließend erhitzte man 2 Stunden unter Rückfluß zum Sieden, engte ein und chromatographierte an Kieselgel. Man erhielt 1,5 g (51 %) 3-Cyano-5-dimethoxymethyl-2-phthalimido-pyrazin vom Festpunkt F = 115 bis 116°C.
$^1$H-NMR (270 MHz, $CDCl_3$): 3,5 (s, 6H), 5,5 (s, 1H), 8,0 (m, 4H), 9,1 (s, 1H).

h) Herstellung von 2-Amino-3-cyano-5-dimethoxymethyl-pyrazin

Zu einer Suspension von 2 g 3-Cyano-5-dimethoxymethyl-2-phthalimido-pyrazin in 40 ml absolutem Methanol wurden bei 40°C 0,2 g Hydrazinhydrat zugetropft. Sofort nach Zugabe klärte sich die Lösung auf und einige Minuten später fielen Kristalle aus. Nach Chromatographie an Kieselgel erhielt man 0,9 g (75 %) 2-Amino-3-cyano-5-dimethoxymethyl-pyrazin.
$^1$H-NMR (270 MHz, DMSO-$d_6$): δ 3,3 (s, 6H), 5,0 (s, 1H), 8,35 (s, 1H).

**Beispiel 2**

Herstellung von 5-Dichlormethyl-3-hydroxymethyl-2-phthalimido-pyrazin direkt aus 3-Chlormethyl-5-dichlormethyl-2-phthalimidopyrazin

Zu einer Lösung von 57,7 g (0,16 mol) 3-Chlormethyl-5-dichlormethyl-2-phthalimino-pyrazin (65 %-ig) in 1000 ml eines Dimethylsulfoxid/Wasser-Gemisches (95 : 5) wurden 50 ml Eisessig und 35 g Kaliumacetat zugegeben. Nach dreitägigem Rühren bei RT setzte man 1000 ml Dichlormethan und 1000 ml Eiswasser zu, durchmischte und trennte die Phasen. Die Wasserphase wurde einmal mit 500 ml und einmal mit 250 ml Dichlormethan, die vereinigten organischen Phasen zweimal mit 1000 ml Wasser gewaschen, über Natriumsulfat getrocknet und eingeengt. Das anfallende schwarze Öl wurde mit 50 ml Methanol zum Kristallisieren gebracht und anschließend aus 700 ml Methanol umkristallisiert. Man erhielt 16,6 g (47 %) 5-Dichlor-methyl-3-hydroxymethyl-2-phthalimidopyrazin mit einem Festpunkt F = 191 bis 192°C (Subl. unter Zers.).

$^1$H-NMR (200 MHz, $CDCl_3$): δ 3,3 (t, OH), 4,8 (d, 2H), 6,9 (s, 1_H), 8,0 (m, 4H), 9,1 (s, 1H).

## Patentansprüche

1. In 3- und 5-Stellung substituierte 2-Amino-pyrazin-derivate der allgemeinen Formel I

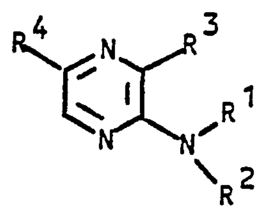

(I),

in der $R^1$ und $R^2$ für Wasserstoff oder eine für Aminogruppen übliche Schutzgruppe stehen, wobei $R^1$ und $R^2$ auch miteinander verbunden sein können,

$R^3$ für eine der Gruppen $-CH_2-O-CO-CH_3$ (a), $-CH_2OH$ (b),

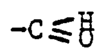

(c), oder $-C\equiv N$ (d) steht und

$R^4$ für $-CHCl_2$ oder $-CHBr_2$ steht oder

$R^4$ für den Fall, daß $R^1$ und $R^2$ für eine Schutzgruppe stehen,

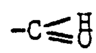

bedeutet.

2. In 3- und 5-Stellung substituierte 2-Amino-pyrazin-derivate der allgemeinen Formel I gemäß Anspruch 1.

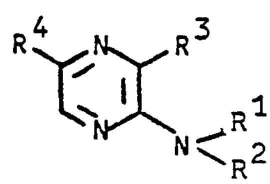

(I),

in der

$R^1$ und $R^2$ zusammen für, die Gruppe

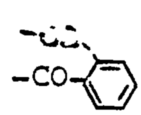

stehen,

$R^3$ für eine der Gruppen $-CH_2-OH$, $-CH_2-O-CO-CH_3$,

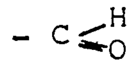

oder -C≡N steht und
$R^4$ für $-CHCl_2$, $-CHBr_2$ oder

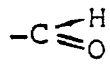

steht.

3. Verfahren zur Herstellung von 2-Amino-3-cyano-5-dialkoxymethyl-pyrazin-derivaten der allgemeinen Formel III

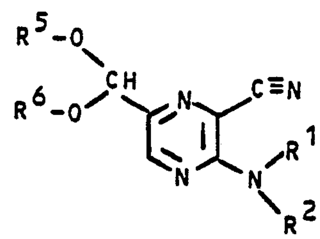

(III),

in der $R^1$ und $R^2$ für Wasserstoff oder eine für Aminogruppen übliche Schutzgruppe stehen, wobei $R^1$ und $R^2$ auch miteinander verbunden sein können
und $R^5$ und $R^6$ für eine Alkylgruppe mit 1 bis 4 C-Atomen stehen, oder aber
$R^5$ und $R^6$ zusammen für einen gegebenenfalls durch Methylgruppen substituierten Ethylen- oder Propylenrest stehen, dadurch gekennzeichnet, daß man

A. ein neues 3-Halogenmethyl-5-dihalogenmethyl-2-amino-pyrazin-derivat der allgemeinen Formel IV

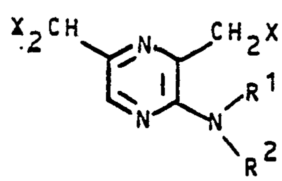

(IV),

in der X für Cl oder Br, vorzugsweise Cl steht, entweder direkt oder über die neue 3-Acetoxymethyl-5-dihalogenmethyl-2-amino-pyrazin-Verbindung der allgemeinen Formel Ia

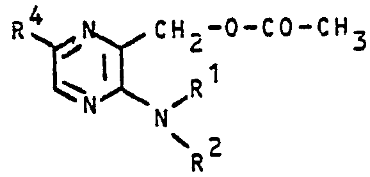

(Ia),

in der $R^1$ und $R^2$ die oben angegebene Bedeutung haben und $R^4$ für $-CHCl_2$ oder $-CHBr_2$, vorzugsweise für $-CHCl_2$ steht,
in das neue 3-Hydroxymethyl-5-dihalogenmethyl-2-amino-pyrazin-derivat der allgemeinen Formel Ib

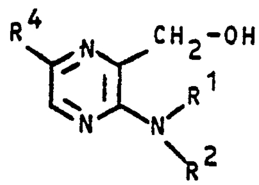

(Ib)

überführt,

B. dieses zu dem entsprechenden neuen 3-Formyl-5-dihalogenmethyl-2-amino-pyrazin-derivat der allgemeinen Formel Ic oxidiert

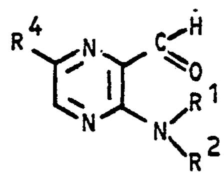

(Ic),

C. dieses in an sich bekannter Weise in das entsprechende neue 3-Cyano-5-dihalogenmethyl-2-amino-pyrazin-derivat der allgemeinen Formel Id überführt,

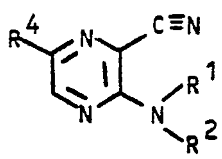

(Id),

D. dieses in an sich bekannter Weise in das entsprechende 3-Cyano-5-formyl-2-amino-pyrazin-derivat der allgemeinen Formel Ie

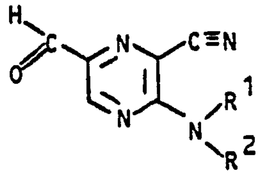

(Ie),

überführt und

E. dieses acetalisiert und gegebenenfalls die Schutzgruppe von der Aminogruppe abspaltet.

4. Verfahren gemäß Anspruch 3, <u>dadurch gekennzeichnet</u>, daß man die Oxidation von Ib zu Ic im Reaktionsschritt B mit einem Pyridiniumchlorochromat in einem Halogenkohlenwasserstoff als Lösungsmittel durchführt.

## Claims

1. A 2-amino-pyrazine derivative which is substituted in the 3- and 5-positions, of the formula I

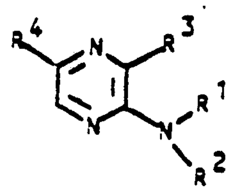

(I)

where $R^1$ and $R^2$ are each hydrogen or a protective group usually employed for amino groups, and may furthermore be bonded to one another,

$R^3$ is $-CH_2-O-CO-CH_3$ (a), $-CH_2OH$ (b),

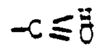

(c) or $-C\equiv N$ (d) and

$R^4$ is $-CHCl_2$ or $-CHBr_2$, or,

where $R^1$ and $R^2$ are each a protective group, $R^4$ may furthermore be

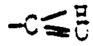 .

2. A 2-amino-pyrazine derivative which is substituted in the 3- and 5-positions, of the formula I as claimed in claim 1

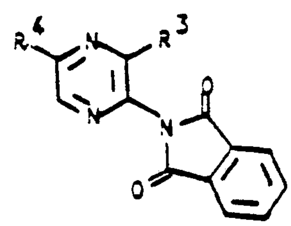

where $R^3$ is $-CH_2-OH$, $-CH_2-O-CO-CH_3$,

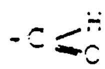

or $-C\equiv N$ and $R^4$ is $-CHCl_2$, $-CHBr_2$ or

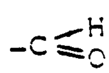 .

3. A process for the preparation of a 2-amino-3-cyano-5-dialkoxymethyl-pyrazine derivative of the formula III

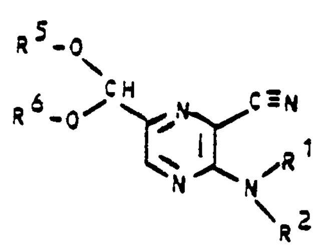

(III)

where $R^1$ and $R^2$ are each hydrogen or a protective group usually employed for amino groups, and may furthermore be bonded to one another, and $R^5$ and $R^6$ are each alkyl of 1 to 4 carbon atoms, or $R^5$ and $R^6$ together form an ethylene or propylene radical which is unsubstituted or substituted by methyl, wherein

A. a novel 3-halomethyl-5-dihalomethyl-2-amino-pyrazine derivative of the formula IV

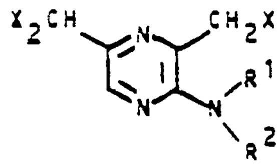

(IV)

where X is Cl or Br, preferably Cl, is converted, either directly or via the novel 3-acetoxymethyl-5-dihalomethyl-2-amino-pyrazine compound of the formula Ia

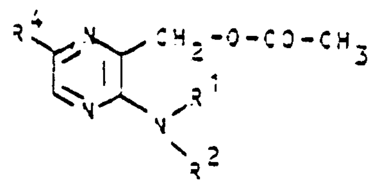

(Ia)

where $R^1$ and $R^2$ have the above meanings and $R^4$ is $-CHCl_2$ or $-CHBr_2$, preferably the former, to the novel 3-hydroxy-methyl-5-dihalomethyl-2-amino-pyrazine derivative of the formula Ib

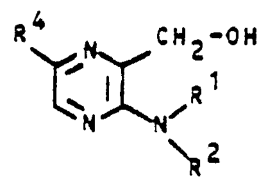

(Ib),

B. this is oxidized to the corresponding novel 3-formyl-5-dihalomethyl-2-amino-pyrazine derivative of the formula Ic

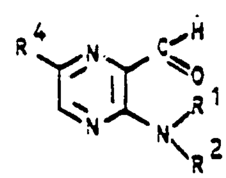

(Ic),

C. the latter is converted in a conventional manner to the corresponding novel 3-cyano-5-dihalomethyl-2-amino-pyrazine derivative of the formula Id

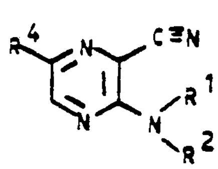

(Id),

D. this is converted in a conventional manner to the corresponding 3-cyano-5-formyl-2-amino-pyrazine derivative of the formula Ie

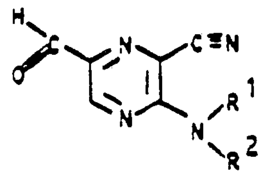

(Ie)

and

E. the latter is acetalized and, if necessary, the protective group is eliminated from the amino group.

4. A process as claimed in claim 3, wherein the oxidation of Ib to Ic in reaction step B is carried out using a pyridinium chlorochromate in a halohydrocarbon as a solvent.

## Revendications

1. Dérivés de 2-amino-pyrazine, substituée en position 3 et 5 de formule générale I

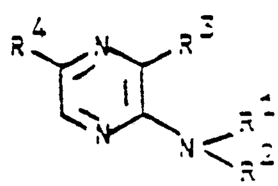

(I),

dans laquelle $R^1$ et $R^2$ sont mis pour hydrogène ou un groupe protecteur usuel pour les groupes amino, $R^1$ et $R^2$ pouvant aussi être liés entre eux,

$R^3$ est mis pour un des groupes $-CH_2-O-CO-CH_3$ (a),

$-CH_2OH$ (b),

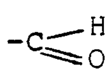

(c), ou $-C\equiv N$ (d) et

$R^4$ est mis pour $-CHCl_2$ ou $-CHBr_2$ ou

$R^4$ représente

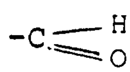

dans le cas où $R^1$ et $R^2$ sont mis pour un groupe protecteur.

2. Dérivés de 2-amino-pyrazine, substituée en position 3 et 5 de formule générale I, selon la revendication 1

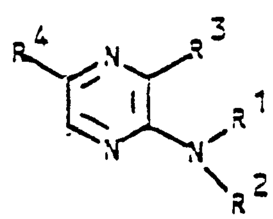

(I),

dans laquelle

$R^1$ et $R^2$ sont mis, ensemble, pour le groupe

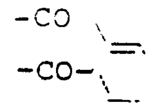

$R^3$ est mis pour un des groupes $-CH_2$-OH, $-CH_2$-O-CO-$CH_3$, -C ou, -C≡N et $R^4$ est mis pour $-CHCl_2$, $-CHBr_2$ ou

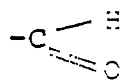

3. Procédé de préparation de dérivés de 2-amino-3-cyano-5-dialcoxyméthyl-pyrazine de formule générale III

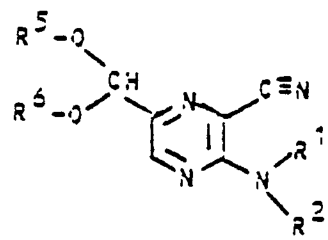

(III),

dans laquelle $R^1$ et $R^2$ sont mis pour hydrogène ou un groupe protecteur usuel pour les groupes amino, $R^1$ et $R^2$ pouvant aussi être liés entre eux,
et $R^5$ et $R^6$ sont mis pour un groupe alkyle de 1 à 4 atomes C, ou bien $R^5$ et $R^6$ sont mis, ensemble, pour un reste éthylène ou propylène éventuellement substitué par des groupes méthyle, caractérisé par le fait que

A. on transforme un nouveau dérivé de 3-halogéneométhyl-5-dihalogénométhyl-2-amino-pyrazine de formule générale IV

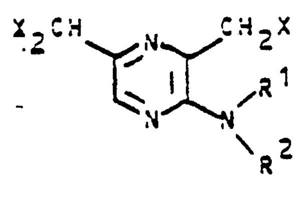

(IV),

dans laquelle X est mis pour Cl ou Br, de préférence Cl, soit directement, soit par l'intermédiaire du nouveau composé 3-acétoxyméthyl-5-dihalogénométhyl-2-amino-pyrazine de la formule générale Ia

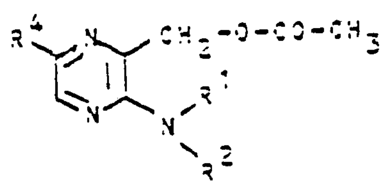

(Ia),

dans laquelle $R^1$ et $R^2$ ont les significations données plus haut et $R^4$ est mis pour $-CHCl_2$ ou $-CHBr_2$, de préférence pour $-CHCl_2$,
en le nouveau dérivé de 3-hydroxyméthyl-5-dihalogéno-méthyl-2-amino-pyrazine de formule générale Ib

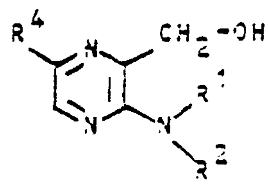

(Ib)

B. on oxyde celui-ci pour le transformer en le nouveau dérivé correspondant de 3-formyl-5-dihalogéno-méthyl-2-amino-pyrazine de formule générale Ic

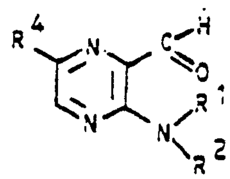

(Ic),

C. on transforme celui-ci, de manière connue en soi, en le nouveau dérivé correspondant de 3-cyano-5-dihalogéno-méthyl-2-amino-pyrazine de formule générale Id

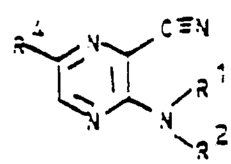

(Id),

D. on transforme celui-ci, de manière connue en soi, en le dérivé correspondant de 3-cyano-5-formyl-2-amino-pyrazine de formule générale Ie

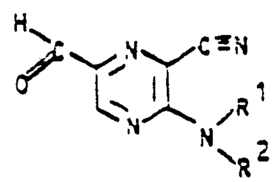

(Ie),

et

E. on acétalise celui-ci et on sépare éventuellement le groupe protecteur du groupe amino.

4. Procédé selon la revendication 3, caractérisé par le fait que l'on effectue l'oxydation de Ib en Ic, dans la phase de réaction B, avec un chlorochromate de pyridinium dans un hydrocarbure halogéné comme solvant.